# EUROPEAN PATENT SPECIFICATION

(11) **EP 1 383 863 B1**
(45) Date of publication and mention of the grant of the patent: **20.02.2008**
(21) Application number: 01271433.3
(22) Date of filing: 19.12.2001
(51) Int. Cl.: C12M 3/00

(54) **TISSUE GROWTH METHOD AND APPARATUS**
GEWEBEKULTUR UND -GERÄT
DISPOSITIF ET METHODE DE CROISSANCE TISSULAIRE

(30) Priority: 20.12.2000 GB 0031089
(43) Date of publication of application: 28.01.2004
(73) Proprietor: UNIVERSITY COLLEGE LONDON, London WC1E 9BT (GB)
(72) Inventor: BROWN, R. A., Medical School, Univ. College London, Greater London W1W 7 EJ (GB); BLUNN, G., Inst. of Orthopaedics and Biomed. Eng., Stanmore, Middlesex HA7 4LP (GB)
(74) Representative: Sutcliffe, Nicholas Robert
(86) International application number: PCT/GB2001/005657
(87) International publication number: WO 2002/050242

(56) References cited:
- WO-A-92/13003
- WO-A-94/25584
- WO-A-98/01810
- US-A- 6 121 042

## Description

The present invention relates to a tissue growth method and apparatus. More particularly, it relates to aspects of long term culture and control of artificially grown tissues.

Cells may be grown in vitro for a function or for study by seeding the cells onto a substrate and providing the cells with the necessary environment and nutrients to grow.

So, for example, US-A-5981211 describes the formation of extracorporeal artificial liver tissue, and V.C. Mudera et al., Cell Motility and the Cytoskeleton, Vol. 45, pp. 1-9, (2000) and R.A. Brown et al., J. of Cellular Physiology, Vol. 175, pp. 323-332, (1998) describe the effects of various loading regimes on the growth of fibroblasts.

However, there is a need to develop further methods for growing tissue in vitro, particularly connective tissues rich in collagen.

In vivo, many cells grow and perform their function in response to specific mechanical cues (many soft tissues and organs exist under predominantly tensile loading). For example, tendon cells experience largely tensile forces. Another example are endothelial cells which grow on the inner surface of blood vessels. This type of cell responds to the shear forces exerted on it by the flow of blood along the blood vessel. On the other hand, cartilage cells grow in vivo under external compression.

Also, in vivo, nutrients for the cells are supplied by microvascular flow, whereas in vitro the nutrients are usually supplied by diffusion from a (typically fluid) culture medium.

US 6 121 042 discloses apparatuses and methods for simulating in vivo conditions while seeding and culturing three-dimensional tissue constructs. Fluid flow is provided by e.g. a pump. WO 94/25584 discloses a cartridge for culturing cells containing hollow fibers. The surface of the cartridge may be coated with fibronectin.

The present invention is at least partly based on the recognition that the appropriate mechanical cues should be applied to cells growing in vitro, and that such cues can also be used to enhance the supply of nutrients to the cells.

Consequently, the present invention provides, in a first aspect, a method for growing tissue in vitro according to claim 1.

Effectively the load is oscillatory and causes a corresponding oscillatory strain in the substrate. The growing cells respond to this strain so that the mechanical cue experienced by the cells can be controlled by controlling the load applied to the substrate (load control) or by controlling the deformation of the substrate (deformation control). In any event, as a result of each load cycle, overall the culture medium is moved along the flow channel, which allows fresh culture medium to be supplied to the growing cells, e.g. via the one-way valve. This is because movement of the culture medium in one direction, corresponding to one half of a load cycle, is inhibited, which leads to a flow of the culture medium in the opposing direction during the other half of the load cycle. In this way, the varying load simultaneously provokes both mechanical cueing and refreshment of the culture medium.

The cells may be for example fibroblasts, chondrocytes, bone cells, endothelial cells, vascular cells, neural cells, epithelial cells, secretary cells, transfected cells, stem cells or muscle cells. As a result of the mechanical cueing, the cells may be induced to produce connective tissue material with specific and useful properties. This material may be collagen, for example, in which the collagen protein filaments are aligned with the direction of substrate deformation. Other connective tissue materials include: fibronectin, fibrin, fibrinogen, vitronectin, laminin, keratin and certain natural polysaccharides such as hyaluronan.

The frequency of load cycling is preferably in the range 0.1 to 60 cycles per hour, and more preferably 0.5-10 cycles per hour. The frequency may vary as the stiffness of the substrate changes. The substrate strain variation (i.e. difference between maximum and minimum substrate elongation divided by the substrate length) caused by each load cycle is preferably in the range 0.05% to 10% and more preferably in the range 0.1% to 1%.

The amount of net deformation experienced by the substrate may also be increased or ramped with time so as to elongate the substrate, i.e. an increasing strain may be superimposed on the cyclical strain. Preferably the net substrate strain is increased at a rate in the range 0.001% to 0.1% per hour, and more preferably in the range 0.005% to 0.05% per hour. Preferably, as a result of increasing the net substrate strain, the total extension (i.e. final deformed length/original length) of the substrate during a complete programme of tissue growth is in the range 1 to 50%, and more preferably in the range 5 to 20%.

In step (ii) the loading regime may be varied in response to a changing mechanical property (e.g. stiffness) of the substrate. For example, as the cells produce tissue the substrate may stiffen. The increasing stiffness may be sensed by an indwelling or remote sensor and used to vary the loading pattern.

The starting substrate may be made from a material such as a synthetic polymer or an aggregated natural macromolecule (protein or polysaccharide, for example). Coatings of natural cell attachment proteins on the substrate may also be used.

Preferably, the substrate is made from aggregated or fibrous fibronectin or from fibronectin-containing composites (e.g. fibronectin-fibrin composites). These substances promote cell attachment, and can be formed as compliant, stretchy material that can sustain substantial elongations. Alternatively or additionally, the substrate may comprise at least one of: fibrin, fibrinogen, laminin, vitronectin-based materials and collagen.

The substrate may be capable of being actively broken down by the introduction of an effective amount of a breakdown agent to the substrate, e.g. via the culture medium. For example the material of the substrate may incorporate targets for the breakdown agent which, when acted upon by the breakdown agent, degrade the substrate into components which may be evacuated by the flow of culture medium. Such targets may be peptide sequences sensitive to specific protease cleavage, so that addition of the appropriate activated protease to the culture medium at a suitable stage of tissue development cleaves the substrate into such components. Alternatively, the peptide sequences may be proenzymes which are activatable to break down the substrate. In any event, this approach can avoid potential problems of adverse, allergic, infective or toxic reactions to the substrate if the tissue is subsequently used for in vivo implantation. For fibronectin-based materials, a suitable proenzyme peptide sequence is plasminogen which is activatable (by an activator such as urokinase plasminogen activator or tissue-type plasminogen activator) to form plasmin which in turn decomposes fibronectin. The same approach can be used with fibrin, fibrinogen, laminin or vitronectin-based materials, and also with some types of collagen.

Non-physiological polymers, recombinant proteins or neoproteins which are capable of passive breakdown (i.e. without the addition of specific agents such as activated proteases) can also be used to form the substrate. Therefore another possible material for the substrate is a non-physiological polypeptide which could be antigenic in vivo, but will normally be fully resorbed before the tissue is used for a medical application.

Synthetic substrate material which is not intended to be broken down before in vivo implantation may include e.g. RGDS cell attachment peptide sequences to promote cell attachment, adhesion and/or migration.

Preferably the substrate has a plurality of parallel flow channels. For example, the substrate may comprise an array or bundle of substantially parallel elongate members aligned substantially parallel to the direction of load application, so that the culture medium moves along the interstices (i.e. flow channels) between the elongate members and diffuses transversely to the members to supply nutrients to the growing cells.

In one embodiment, the elongate members are substantially solid fibres (e.g. of fibronectin), with cell growth occurring on the surfaces of the fibres. These fibres may have, for example, diameters in the range 5-500 µm.

In another embodiment the elongate members comprise a substantially solid core (e.g. of fibronectin) and a relatively compliant gel coating (e.g. of fibrin, collagen, proteoglycan, glycosaminoglycan such as hyaluronan, polysaccharide or synthetic bioresorbable polymer such as polylactic acid, polyglycolic acid and polycapriolactone). The overall diameter of the core plus gel coating may again be in the range 5-500 µm, but the solid core may have a diameter of only 20-50 nm. Cell growth may occur on the surface of and/or within the gel coating. The gel coating increases the overall compliance of the substrate and thereby allows greater substrate deformations. This may in turn increase the flow of culture medium along the interstices between the elongate members.

In another embodiment, the elongate members are fine tubes (as described e.g. in S.I. Harding et al., "The Scaleable Preparation of Fibronectin-Based Tubes, Suitable as Tissue Engineering Scaffolds", 1st NELSIC meeting, UCL, London, Sept 2000, and S.I. Harding, PhD thesis, University College London, 1999). Here, the culture medium may also move along the insides of the tubes, and cell growth may occur on the inside surfaces and/or the outside surfaces of the tubes. Typically, these tubular elongate members are formed of fibronectin, fibrin and/or fibrinogen. They may have an internal diameter of 0.1-2 mm, preferably 0.1-1 mm. The wall thickness may be in the range 5-250 µm.

The substrate may further comprise a mixture of elongate members of different type (e.g. a mixture of any two or three of the above embodiments).

In vivo, mass transfer from the vascular system to cells across distances greater than about 0.4 mm is insufficient to support many cell types, and similarly nutrients in vitro generally cannot perfuse sufficiently through tissue thicknesses greater than about 0.4 mm to support cells and cell growth. Nonetheless, some medical applications call for artificially grown tissue to be of greater thickness than this, and by providing a substrate which is an array or bundle of elongate members it is possible to grow relatively dense tissue to sizes significantly greater than the maximum perfusion depth. Essentially, the multiple interstitial flow channels for the culture medium formed by the array or bundle mimic in vivo vascular systems, so that although each flow channel may only support perfusion up to a radial distance of 0.4 mm from the channel, in combination the channels can supply significantly larger tissue growth thicknesses with nutrients.

In a second aspect, the present invention provides a tissue growth apparatus according to claim 9.

The apparatus of the second aspect may be used to perform the method of the first aspect, and may further include preferred optional features described in respect of the first aspect.

The attachment means may comprise e.g. a mechanical, magnetic or adhesive fastener for connecting the substrate to the load application means. Alternatively the attachment means may merely hold the substrate relative to the apparatus, and the substrate may be fitted with magnetic end pieces which are remotely movable by an externally applied electromagnetic field to load the substrate. The load application means may be a computer-controlled device such as an electric motor. However, it may also be a hydraulic drive. Alternatively, the contractions of exogenously controlled contractile cells or the flow of fluid through and/or around the substrate may be harnessed to apply the load.

The load application means may comprise sensing means for sensing a changing mechanical property (e.g. stiffness) of the substrate and control means for varying the loading regime in response to the changing mechanical property sensed by the sensing means. The sensing means may be e.g. an indwelling or remote sensor for the substrate.

Preferred embodiments of the invention will now be described, by way of example only, with respect to the accompanying drawings, in which:
Fig. 1 shows a schematic drawing of a tissue growth apparatus,
Fig. 2 shows a schematic transverse view of a fibre seeded with cells at the beginning of a tissue growth programme,
Fig. 3 shows a schematic transverse view of the fibre of Fig. 2 after straining with tissue growing around the fibre,
Fig. 4 shows a schematic transverse view of another form of fibre, and
Fig. 5 shows a schematic transverse view of yet another form of fibre.

For the artificial growth of connective tissues, it can be important to provide meaningful mechanical control cues so as to grow tissue which mimics as nearly as possible natural tissue. The local micro-mechanical environment of each cell which is incorporated in the tissue influences the cell=s responsiveness to chemical mediators, the cell=s morphology and behaviour, and ultimately the matrix of corrective tissue which the cell produces.

For example, fibroblasts and smooth muscle cells respond to an applied strain by producing collagen which is anisotropic in the sense that the collagen protein and fibril/fibre strands are aligned in the direction of the strain. This relieves the cells from stress, i.e. stress due to the applied strain is taken up by the collagen, so that further collagen produced by the cells will not be aligned unless a further strain is applied.

Consequently, application of a static strain to a seeded substrate only results in alignment of the initial tissue grown. Subsequent tissue grown may not be similarly aligned unless the strain applied to the substrate is varied.

Another important factor is the supply of nutrients to (and removal of waste products from) the cells which grow the tissue. We have found that this supply can be promoted by applying a cyclically varying strain (via a cyclically varying load) to the substrate on which the cells are grown, whereby the applied load serves a dual purpose of providing mechanical cueing and refreshing the culture medium which supplies nutrients to the cells.

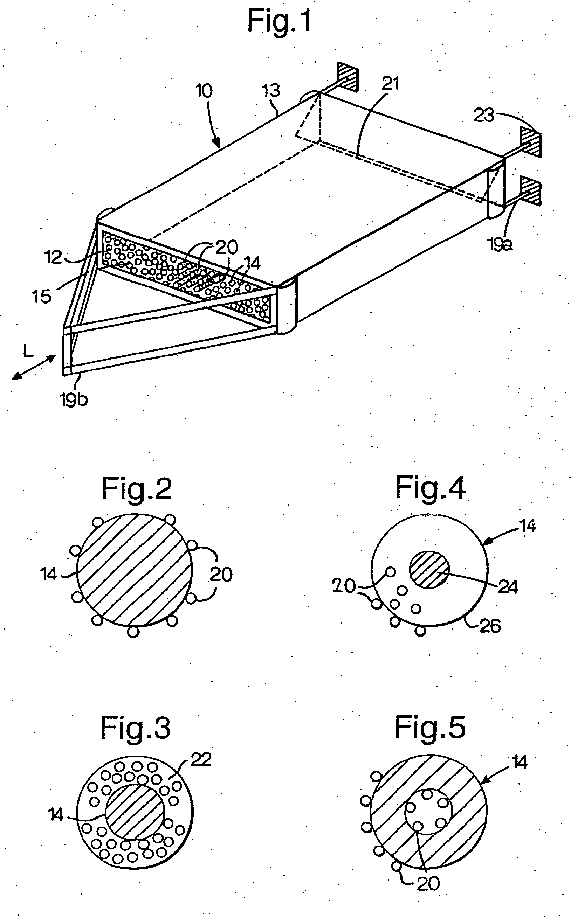

Fig. 1 shows a schematic drawing of part of an apparatus 10 according to the present invention for growing tissue in vitro. Fibres 14, seeded with fibroblast cells 20, are arranged parallel to each other in a substrate bundle 12 (which is here shown with a rectangular cross-section, but other cross-sections, e.g. circular, may be used), and held together by outer casing 13 which is of e.g. collagen, fibronectin, fibrin, fibrinogen or synthetic polymer. The bundle has longitudinally extending parallel interstitial gaps 15 which serve as flow channels, the gaps being formed by the packing arrangement of the circular cross-sectioned fibres 14. The apparatus may contain a plurality of such substrate bundles.

The casing is removably fixed at one end by a mechanical fastener 19a to the body 23 (indicated only schematically in Fig. 1) of the apparatus. The other end of the casing is removably attached by a fastener 19b to a loader (not shown) which applies a cyclical load, L, to the bundle of fibres, the load being transmitted to the bundle via the casing. The loader may be a computer-controlled electric motor.

The cyclical load produces a ramped cyclical strain in the fibre bundle. The frequency of the cyclical strain is about 1 cycle per hour, and the strain has an amplitude of about 0.5% per cycle. The maximum strain in adjacent cycles increases by about 0.001%.

Supply means (not shown, but which may comprise a liquid culture medium reservoir and a supply conduit for transporting the culture medium to the fixed end of the casing) supplies the culture medium to the fixed end of the casing where a flap valve 21 allows the culture medium to enter the casing but not to return in the opposite direction. Culture medium which has passed through bundle 12 is discharged from the other (moving) end of the casing.

We believe that the cyclical deformation of the substrate bundle imposes a corresponding pressure variation in the culture medium contained in the gaps 15. When the pressure is relatively high, flap valve 21 largely prevents the culture medium from exiting from the fixed end of the casing and culture medium is therefore discharged from the moving end of the casing. Conversely, when the pressure is relatively low flap valve 21 permits entry of fresh culture medium from the supply means. So overall there is a net flow of culture medium along the gaps from the fixed end to the moving end of the casing, fresh culture medium being supplied via the flap valve to the substrate bundle.

Because the fibres are relatively narrow (compared to the size of the complete bundle) and each carries only a small proportion of the total number of growing cells, the culture medium is able to perfuse transversely to substantially all of the cells growing on the substrate. Essentially, the construction of the fibre bundle forms an array of parallel "capillaries" along which the culture medium can flow. The pressure variation in the culture medium probably also helps to enhance transverse perfusion to the cells. The "capillary network" closely mimics natural tissue which provides an expectation that tissue formed in this way should integrate well when implanted in vivo. That is, host blood capillaries and nerve cells may grow preferentially along the capillaries.

Substrate bundle 12 and casing 13 may be entirely or partially immersed in liquid culture medium. This can help to support the substrate bundle, and, if the casing is permeable, also help to supply the substrate with nutrients.

Each fibre 14 has a diameter of about 100-200 µm, and is made from fibronectin, a material to which many types of cell can attach and adhere. Fibronectin fibres are also capable of deforming to many times their original length, as discussed in e.g. our earlier WO 92/13003.

Generally it is found that the nutrients needed for cell growth cannot be delivered through a thickness of tissue of more than about 0.4 to 1 mm, depending on e.g. the density of the tissue, cell metabolic activity etc. Therefore, if the cells were grown on, for example, a flat substrate, the maximum possible thickness of tissue which could be obtained with healthy, mature cells would be about 0.4 to 1 mm. In the present invention, the provision of flow channels throughout the substrate means that a total tissue thickness which is significantly greater than 0.4 to 1 mm can be achieved.

The fibronectin fibres are resorbable and during the tissue growth programme, the fibronectin is gradually resorbed. Consequently, the growing tissue takes up more and more of the applied load originally carried by the fibre as the fibronectin resorbs. When all of the fibronectin has resorbed the tissue takes up all of the applied load.

Fig. 2 shows a schematic transverse view of a fibre 14 seeded with cells 20 at the start of a tissue growth programme. Fig. 3 shows a schematic transverse view of the same fibre 14 later in the tissue growth programme. The fibre has been reduced in cross sectional area because it has been elongated by the strain and also been resorbed. The cells have multiplied and produced an extracellular matrix of collagen connective tissue 22.

Fig. 4 shows a cross-sectional view of an alternative form of fibre for forming the substrate bundle, in which a solid core 24 of fibronectin is surrounded by a relatively compliant fibrin or collagen gel coating 26. The overall diameter of the core plus gel coating is about 250 µm, but the solid core has a diameter of about only 40 nm. Cell growth may occur on the surface of and within the gel coating.

Fig. 5 shows a cross-sectional view of a tubular fibronectin fibre which can also be used to form the substrate bundle. This fibre has an internal diameter of about 0.2 mm and a wall thickness of about 100 µm. With this type of fibre the culture medium can flow along the inside of the tubes as well as along the interstitial gaps between the tubular fibres of the substrate bundle. Cell growth can occur on the inside and outside surfaces of the tubular fibres.

The substrate bundle can also be formed from a mixture of these types of fibre, the relative number and position in the substrate bundle of each type being optimised to provide the desired substrate characteristics. For example, relatively wide diameter solid fibronectin fibres (Fig. 2) stiffen the matrix and provide wide interstitial gaps, gel coated fibres (Fig. 4) provide enhanced cell seeding levels and improved perfusion, and tubular fibres (Fig. 5) improve early stage perfusion, particularly to deeper regions of the substrate bundle.

Whilst various embodiments of the present invention have been described in detail, modifications and adaptations of these and further embodiments will be apparent to those skilled in the art. However, it is to be expressly understood that such further embodiments are within the scope of the present invention.

## Claims

1. A method for growing tissue in vitro including the steps of:
(i) providing a deformable substrate (12) which is seeded with tissue-forming cells (20) and which defines at least one flow channel (15) containing a fluid culture medium;
(ii) applying, substantially parallel to the flow channel, a cyclically varying load (L) to the substrate, which load deforms the substrate to provide mechanical cueing for the cells; and
(iii) inhibiting the flow of culture medium in one direction of the flow channel;
**characterised in that** the deformation of the substrate causes a net flow of the culture medium along the flow channel in the opposing direction, thereby refreshing the culture medium in the flow channel.

2. A method according to claim 1, wherein the frequency of the load cycling is in the range 0.1 to 60 cycles per hour.

3. A method according to claim 1 or 2, wherein the substrate strain variation, being the difference between the maximum and minimum substrate elongation divided by the substrate length, per load cycle is in the range 0.05% to 1%.

4. A method according to any one of the preceding claims, wherein the net substrate strain is increased at a rate in the range 0.001% to 0.1% per hour.

5. A method according to any one of the preceding claims, wherein the substrate comprises fibronectin.

6. A method according to any one of the preceding claims, including the further step of:
(iv) breaking down the substrate by introducing an effective amount of a breakdown agent to the substrate.

7. A method according to any one of the preceding claims, wherein the substrate defines a plurality of flow channels.

8. A method according to claim 7, wherein the substrate comprises a bundle of substantially parallel elongate members (14) aligned substantially parallel to the direction of load application, the flow channels being formed by the interstices between the elongate members.

9. A tissue growth apparatus (10) including:
(i) attachment means (19a, 19b) which removably attaches to the apparatus a deformable substrate which defines at least one flow channel (15) and is seeded with tissue-forming cells, in use the flow channel containing fluid culture medium;
(ii) fluid supply means for supplying fresh fluid culture medium to the flow channel;
(iii) load application means for applying, substantially parallel to the flow channel, a cyclically variable load (L) to the substrate, which load, in use, deforms the substrate to provide mechanical cueing for the cells; and
(iv) one-way valve means (21) for inhibiting the flow of culture medium in one direction of the flow channel;
**characterised in that** the one-way valve means and the deformable substrate are arranged such that deformation of the substrate causes a net flow of the culture medium along the flow channel in the opposite direction thereby drawing fresh culture medium from the fluid supply means into the flow channel.

## Patentansprüche

1. Verfahren zum In-Vitro-Züchten von Gewebe, umfassend die folgenden Schritte des:
(i) Bereitstellens eines verformbaren Substrats (12), das mit Gewebe ausbildenden Zellen (20) geimpft ist und das zumindest einen Strömungskanal (15) definiert, der ein Kulturfluidmedium enthält,
(ii) Anlegens einer sich periodisch ändernden Last (L) an das Substrat im Wesentlichen parallel zum Strömungskanal, weiche Last das Substrat verformt, um ein mechanisches Auslösesignal für die Zellen bereitzustellen und
(iii) Blockierens des Stroms des Kulturmediums in einer Richtung des Strömungskanals,
**dadurch gekennzeichnet, dass** die Verformung des Substrats eine Nettoströmung des Kulturmediums entlang des Strömungskanals in entgegengesetzter Richtung verursacht, wodurch das Kulturmedium im Strömungskanal erneuert wird.

2. Verfahren nach Anspruch 1, worin die Frequenz der Wechsellast im Bereich von 0,1 bis 60 Zyklen pro Stunde ist.

3. Verfahren nach Anspruch 1 oder 2, worin die Substratdehnungsänderung, welche die Differenz zwischen der maximalen und der minimalen Substratverlängerung geteilt durch die Substratlänge ist, pro Lastzyklus im Bereich von 0,05 % bis 1 % ist.

4. Verfahren nach einem der vorangegangenen Ansprüche, worin die Nettosubstratdehnung mit einer Rate im Bereich von 0,001 % bis 0,1 % pro Stunde ansteigt.

5. Verfahren nach einem der vorangegangenen Ansprüche, worin das Substrat Fibronectin umfasst.

6. Verfahren nach einem der vorangegangenen Ansprüche, umfassend die folgenden Schritte des:
(iv) Zerlegens des Substrats durch Einführen einer wirksamen Menge eines Zerlegungsmittels ins Substrat.

7. Verfahren nach einem der vorangegangenen Ansprüche, worin das Substrat eine Vielzahl an Strömungskanälen definiert.

8. Verfahren nach Anspruch 7, worin das Substrat ein Bündel im Wesentlichen paralleler länglicher Elemente (14) umfasst, die im Wesentlichen parallel zur Richtung des Lastanlegens ausgerichtet sind, wobei die Strömungskanäle durch die Zwischenräume zwischen den länglichen Elementen ausgebildet sind.

9. Gewebewachstumsvorrichtung (10), umfassend:
(i) ein Befestigungsmittel (19a, 19b), das an die Vorrichtung ein verformbares Substrat ablösbar anbringt, welches zumindest einen Strömungskanal (15) definiert und mit Gewebe ausbildenden Zellen geimpft wird, wobei bei Verwendung der Strömungskanal ein Kulturfluidmedium enthält,
(ii) ein Fluidzufuhrmittel zum Zuführen frischen Kulturfluidmediums in den Strömungskanal,
(iii) ein Lastanlegemittel zum im Wesentlichen zum Strömungskanal parallelen Anlegen einer zyklisch änderbaren Last (L) an das Substrat, dessen Last - bei Verwendung - das Substrat verformt, um ein mechanisches Auslösesignal für die Zellen bereitzustellen und
(iv) ein Einwegventilmittel (21) zum Blockieren des Kulturmediumstroms in einer Richtung des Strömungskanals,
**dadurch gekennzeichnet, dass** das Einwegventilmittel und das verformbare Substrat so angeordnet sind, dass die Verformung des Substrats eine Nettoströmung des Kulturmediums entlang des Strömungskanals in der entgegengesetzten Richtung hervorruft, wodurch frisches Kulturmedium aus dem Fluidzufuhrmittel in den Strömungskanal gesaugt wird.

## Revendications

1. Méthode de croissance tissulaire in vitro comprenant les étapes de:
(i) prévoir un substrat déformable (12) qui est ensemencé de cellules (20) formant du tissu et qui définit au moins un canal d'écoulement (15) contenant un milieu de culture fluide;
(ii) appliquer, sensiblement parallèlement au canal d'écoulement, une charge cycliquement variable (L) au substrat, laquelle charge déforme le substrat pour produire une insertion mécanique pour les cellules; et
(iii) inhiber l'écoulement du milieu de culture dans une direction du canal d'écoulement;
**caractérisée en ce que** la déformation du substrat provoque un écoulement net du milieu de culture le long du canal d'écoulement dans la direction opposée, rafraîchissant ainsi le milieu de culture dans le canal d'écoulement.

2. Méthode selon la revendication 1, où la fréquence du cycle de charge est de l'ordre de 0,1 à 60 cycles par heure.

3. Méthode selon la revendication 1 ou 2, où la variation de contrainte du substrat, étant la différence entre l'allongement maximum et l'allongement minimum du substrat divisé par la longueur du substrat, par cycle de charge, est de l'ordre de 0,05% à 1%.

4. Méthode selon l'une quelconque des revendications précédentes, où la contrainte nette du substrat est augmentée à raison de 0,001% à 0,1% par heure.

5. Méthode selon l'une quelconque des revendications précédentes, où le substrat comprend de la fibronectine.

6. Méthode selon l'une quelconque des revendications précédentes, comprenant l'étape supplémentaire de:
(iv) rompre le substrat par introduction d'une quantité efficace d'un agent de rupture dans le substrat.

7. Méthode selon l'une quelconque des revendications précédentes, où le substrat définit un certain nombre de canaux d'écoulement.

8. Méthode selon la revendication 7, où le substrat comprend un faisceau d'organes allongés sensiblement parallèles (14) qui sont alignés sensiblement parallèlement à la direction de l'application de la charge, les canaux d'écoulement étant formés par les interstices entre les organes allongés.

9. Appareil de croissance tissulaire (10) comprenant:
(i) un moyen d'attachement (19a, 19b) qui attache de façon amovible à l'appareil un substrat déformable qui définit au moins un canal d'écoulement (15) et qui est ensemencé de cellules formant du tissu, en utilisation le canal d'écoulement contenant un milieu de culture fluide;
(ii) un moyen d'alimentation en fluide pour fournir du milieu de culture fluide frais au canal d'écoulement;
(iii)un moyen d'a pplication d'une charge pour appliquer, sensiblement parallèlement au canal d'écoulement, une charge cycliquement variable (L) au substrat, laquelle charge, en utilisation, déforme le substrat pour permettre une insertion mécanique pour les cellules; et
(iv) un moyen formant vanne à une voie (21) pour inhiber l'écoulement du milieu de culture dans une direction du canal d'écoulement;
**caractérisé en ce que** le moyen formant vanne à une voie et le substrat déformable sont agencés de façon que la déformation du substrat provoque un écoulement net du milieu de culture le long du canal d'écoulement en direction opposée pour ainsi attirer du milieu de culture frais du moy en d'alimentation en fluide vers le canal d'écoulement.
